# EUROPEAN PATENT SPECIFICATION

(11) **EP 3 128 924 B1**
(45) Date of publication and mention of the grant of the patent: **10.06.2020**
(21) Application number: 15724761.0
(22) Date of filing: 02.04.2015
(51) Int. Cl.: A61B 8/08, A61B 8/00, A61M 25/01, A61B 90/00, A61B 17/34

(54) **SIGNAL VERSUS NOISE DISCRIMINATION NEEDLE WITH PIEZOELECTRIC POLYMER SENSORS**
NADEL ZUR UNTERSCHEIDUNG VON SIGNAL UND RAUSCHEN MIT PIEZOELEKTRISCHEN POLYMERSENSOREN
AIGUILLE DE DISCRIMINATION DE SIGNAL / BRUIT AVEC CAPTEURS EN POLYMÈRE PIÉZOÉLECTRIQUE

(30) Priority: 11.04.2014 US 201461978198 P
(43) Date of publication of application: 15.02.2017
(73) Proprietor: Koninklijke Philips N.V., 5656 AG Eindhoven (NL)
(72) Inventor: ERKAMP, Ramon Quido, 5656 AE Eindhoven (NL); JAIN, Ameet Kumar, 5656 AE Eindhoven (NL); VIGNON, Francois Guy Gerard Marie, 5656 AE Eindhoven (NL); BHARAT, Shyam, 5656 AE Eindhoven (NL)
(74) Representative: Philips Intellectual Property & Standards
(86) International application number: PCT/IB2015/052431
(87) International publication number: WO 2015/155649

(56) References cited:
- WO-A1-98/39668
- US-B1- 6 217 518

## Description

### BACKGROUND:

### Technical Field

This disclosure relates to medical instruments and more particularly to a system and method to track an instrument under ultrasound guidance using ultrasound receivers formed on the instrument that can discriminate between signal and noise.

### Description of the Related Art

In ultrasound imaging, the visibility of the needle is often very poor due to the specular nature of the needle surface that reflects beams away from the imaging probe. To alleviate this problem some needle manufacturers have produced needles with special echogenic coatings, but the visualization improvement is limited. Ultrasound imaging system manufacturers have developed algorithms that use multiple imaging beams from varied angles, but improvement is limited and such a strategy is primarily suited only for linear arrays. Both strategies do not help when the needle is inserted perpendicular to the imaging plane or the needle path has a small offset relative to the imaging plane.

One solution that has been proposed to visualize the tip of interventional tools such as needles, but also catheters, is to add ultrasound receivers near the tip of the tool. While the imaging beam sweeps the field of view, the signals from the sensors indicate how close the beams are getting to the sensor. This information is used to calculate sensor position relative to the ultrasound image with positional accuracy exceeding 0.5 mm, even under conditions where the needle is not visible in the ultrasound image. The sensor needs to not interfere with the functionality of the device (e.g., an automatic biopsy device), that is, not block the lumen, not interfere with the mechanics, etc.

Document US 6,217,518 B1 discloses an ultrasound transducer that includes a sheath body constructed from a nonconductive material. The sheath body is configured to be installed over a medical device. A piezoelectric copolymer transducer is held snugly against the medical device by the sheath body when the ultrasound transducer is installed over the medical device.

### SUMMARY

In accordance with the present principles, a medical device includes a device body, a first sensor formed on the device body and including a piezoelectric polymer as a sensor element, the piezoelectric polymer configured to receive ultrasonic energy and a first electrical trace connecting to the first sensor element and extending along the device body. A dummy sensor is formed on the device body in proximity of the first sensor and includes a dummy sensor element and a second electrical trace connected to the dummy sensor element and extending along the device body in a configuration relative to the first electrical trace, wherein a signal event is discriminated between a signal and noise based on a comparison between a signal strength measured on the first sensor and the dummy sensor.

Another medical device includes a needle, a first ring sensor conformally formed on
the needle and including a piezoelectric polymer as a sensor element, the piezoelectric polymer configured to receive ultrasonic energy and a first dielectric layer formed on the needle. A first electrical trace connects to the first sensor element and extends longitudinally along the needle on the first dielectric layer. A dummy sensor is formed on the needle in proximity of the first sensor and includes a dummy sensor element. The dummy sensor includes a same structure as the sensor. A second electrical trace connects to the dummy sensor element and extends longitudinally along the needle parallel to the first electrical trace, wherein a signal event is discriminated between a signal and noise using a response measured on the first sensor, or the dummy sensor.

A method for discriminating between signal and noise measured by a medical device includes providing a medical device having a device body; at least one first sensor formed on the device body and including a piezoelectric polymer as a sensor element, the piezoelectric polymer configured to receive ultrasonic energy; the at least one first sensor comprising at least one first electrical trace connecting to the first sensor element and extending along the device body, at least one dummy sensor formed on the device body in proximity of the at least one first sensor and including a dummy sensor element; the at least one dummy sensor comprising at least one second electrical trace connecting to the dummy sensor element and extending along the device body in a configuration relative to the at least one first electrical trace; comparing signal strength measured for first sensors and dummy sensors to determine whether a candidate signal is signal or noise; and discarding noise signals.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1A is a perspective view showing a needle having a low profile conformal sensor and a dummy sensor having a similar structure in accordance with exemplary embodiments of the present invention;
FIG. 1B is a cross-sectional view taken at section line 1B-1B of FIG. 1A;
FIG. 1C is a cross-sectional view taken at section line 1C-1C of FIG. 1A;
FIG. ID is a cross-sectional view taken at section line ID-ID of FIG. 1A;
FIG. 2 is a side view showing a needle having a multiple devices including multiple sensors or sensor/dummy sensor pairs in accordance with another embodiment;
FIG. 3 is a side view showing a needle having a dummy wire and/or a dummy device in an attachment cable in accordance with another embodiment;
FIG. 4 is a flow diagram showing a method for discriminating between signal and noise measured by a medical device in accordance with the present principles;
FIG. 5 is a block/flow diagram showing a system/method for isolating relevant sensor signals resulting from one imaging frame for estimating a needle orientation, for the case of a needle with a multitude of sensors, dummy sensors, and dummy traces in accordance with the present principles; and
FIG. 6 is a block/flow diagram showing an iterative procedure for a tracking method for refining the needle (or device) position or determining that the needle position estimate in accordance with the present principles.

### DETAILED DESCRIPTION OF EMBODIMENTS

In accordance with the present principles, systems, devices and methods are provided for tracking a needle (or other device) under ultrasound guidance by attaching small ultrasound receivers onto the device. The present principles provide a needle, device or system that includes one or more low profile sensors at very low per device cost and permits scaling for mass production to maintain low cost. The low profile sensors, in accordance with the present principles, pick up ultrasound induced signals but are less prone to picking up signals produced by external radio frequency (RF) sources. For tracking, the maximum ultrasound generated signal within an ultrasound frame needs to be detected. The present embodiments permit the discrimination of strong signals induced by ultrasound from other strong signals induced by outside influences.

In one embodiment, ultrasound sensors on a needle are fabricated using a piezoelectric polymer. Since the sensors are high impedance, the sensors would otherwise be prone to picking up noise from outside RF fields. The noise is either picked up by the sensor itself or by a trace that connects the sensor to an amplifier. To discriminate this noise, another structure is provided that strongly resembles the geometry of the sensor, and is in close proximity to the sensor and interconnect. For example, another sensor structure may be formed adjacent to the actual sensor, but not polarize it so it will not be sensitive to ultrasound. This "dummy sensor" could then be connected with a trace adjacent to the sensor trace and electrode ring adjacent to the sensor electrode ring. This dummy structure would have similar sensitivity to RF noise sources. During signal processing, if only a strong signal on the sensor is observed but not on the "dummy structure", it indicates a genuine acoustic event. If a strong signal is observed on both structures, it is likely due to noise and disregarded.

In accordance with the present principles, piezoelectric polymers such as, e.g., polyvinylidene fluoride (PVDF) or polyvinylidene fluoride trifluoroethylene (P(VDF-TrFE)). P(VDF-TrFE) are good candidate materials for sensor production. Sensors produced using these polymers can have low signal levels and be prone to picking up RF noise from non-acoustic events. The present methods of discriminating outside noise signals from acoustically generated signals can greatly enhance robustness of tracking using these sensors. Discriminating outside noise signals from relevant acoustic events is provided by low form factor features on the device.

To keep product cost down, the materials employed need to be low cost, and the manufacturing process should be highly automated with large volume to avoid labor and equipment costs. The ultrasound sensors may be formed on the needle or other device and may be fabricated using a piezoelectric polymer, e.g., polyvinylidene fluoride (PVDF) or polyvinylidene fluoride trifluoroethylene (P(VDF-TrFE)). P(VDF-TrFE), which can be dissolved in acetone and applied to the needle through an evaporative process. The sensors are high impedance and can be modeled as a voltage source in series with a small capacitor (e.g., 2.2pF). Such a sensor is very sensitive to capacitive loading of the electrical interconnect, and special capacitance cancelling electronics (similar to, e.g., a driven shield technique) can be employed to avoid large signal loss. A wire carrying the signal preferably is shielded (e.g., includes an electric shield around the conductor).

It should be understood that the present invention will be described in terms of medical instruments; however, the teachings of the present invention are much broader and are applicable to any instrument that can accept a low profile sensor. In some embodiments, the present principles are employed in tracking or analyzing complex biological or mechanical systems. In particular, the present principles are applicable to internal tracking procedures of biological systems and are applicable for procedures in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

It will also be understood that when an element such as a layer, region or material is referred to as being "on" or "over" another element, it can be directly on the other element or intervening elements may also be present. In contrast, when an element is referred to as being "directly on" or "directly over" another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present.

Reference in the specification to "one embodiment" or "an embodiment" of the present principles, as well as other variations thereof, means that a particular feature, structure, characteristic, and so forth described in connection with the embodiment is included in at least one embodiment of the present principles. Thus, the appearances of the phrase "in one embodiment" or "in an embodiment", as well any other variations, appearing in various places throughout the specification are not necessarily all referring to the same embodiment.

It is to be appreciated that the use of any of the following "/", "and/or", and "at least one of', for example, in the cases of "A/B", "A and/or B" and "at least one of A and B", is intended to encompass the selection of the first listed option (A) only, or the selection of the second listed option (B) only, or the selection of both options (A and B). As a further example, in the cases of "A, B, and/or C" and "at least one of A, B, and C", such phrasing is intended to encompass the selection of the first listed option (A) only, or the selection of the second listed option (B) only, or the selection of the third listed option (C) only, or the selection of the first and the second listed options (A and B) only, or the selection of the first and third listed options (A and C) only, or the selection of the second and third listed options (B and C) only, or the selection of all three options (A and B and C). This may be extended, as readily apparent by one of ordinary skill in this and related arts, for as many items listed.

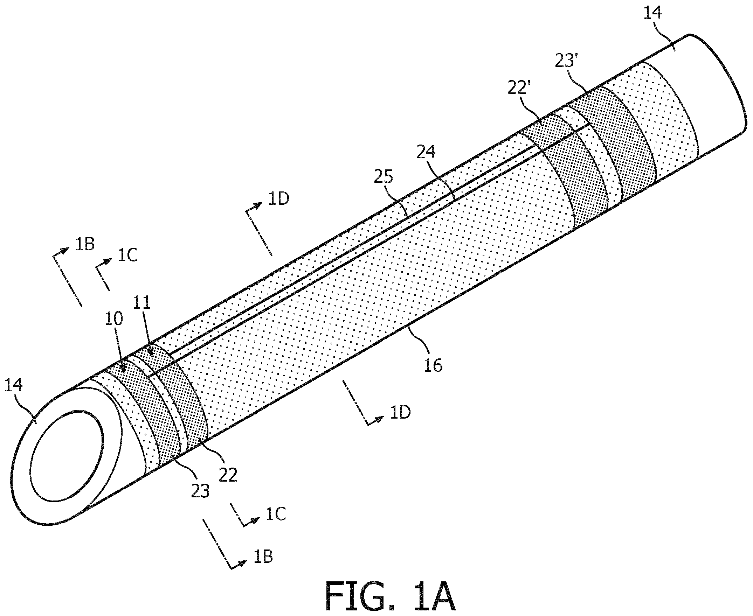

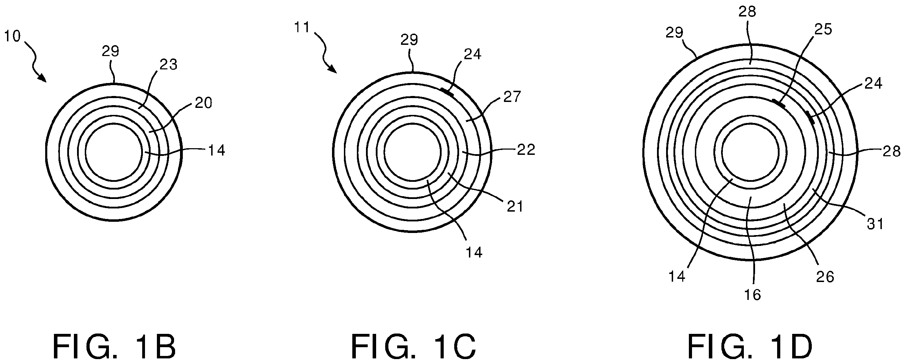

Referring now to the drawings in which like numerals represent the same or similar elements and initially to FIGS. 1A, 1B and 1C, a perspective view (FIG. 1A) (for illustrative purposes the length of a needle 14 has been shortened), a cross-sectional view taken at section line 1B-1B (FIG. 1B), a cross-sectional view taken at section line 1C-1C (FIG. 1C), and a cross-sectional view taken at section line ID-ID (FIG. ID) show the fabricating of a single ring sensor 10 on the needle 14 in accordance with one embodiment. The needle 14 preferably includes a metal, such as a stainless steel although other surgically compatible materials may be employed. An insulator 16 is deposited or printed on the needle 14. The insulator 16 may include any suitable dielectric material that adheres to the needle 14. The insulator 16 may be about 25 - 50 microns thick although other thicknesses may be employed. The insulator 16 is deposited on the needle 14 without covering a small section at the tip region. This may be accomplished in a plurality of ways. For example, the portion may be etched away or the tip may be dip coated at the proximal end of the needle 14, or the distal end may be dipped and a mask is removed to leave an exposed portion.

The tip portion (distal end portion) of the needle 14 is coated with a piezoelectric copolymer 20. This may be achieved by employing a dip coating process. Special care needs to be taken that the copolymer 20 touches or slightly overlaps the insulator layer 16 such that the needle surface is not exposed in the small section exposed through the dielectric layer 16. The metal needle 14 now serves as a bottom electrode for the copolymer sensor 10.

In one embodiment, the copolymer includes a P(VDF-TrFE) ring, although other suitable materials may be employed.

A second section is opened up in the dielectric layer 16 for the formation of a dummy sensor 11. The dummy sensor 11 may include a non-poled version of the copolymer layer 20 forming a non-poled piezoelectric polymer layer or non-piezoelectric layer 21.

A top electrode 22, pad structure 22' and a signal trace 25, connecting the top electrode 22 to the pad structure 22', are formed to create the dummy sensor 11. The top electrode 22 is formed over the copolymer 21 at the distal end portion of the needle 14 (the needle forms the bottom electrode). The trace 25 and pad structure 22' are formed over a portion of the insulator 16 extending toward the proximal end portion of the needle 14. The top electrode 22 and pad structure 22' are connected by the trace 25. The top electrode 22, pad structure 22' and the trace 25 may be printed using a conductive ink. Other processes may be employed as well, such as, e.g., masked vapor deposition or vapor deposition and etching. The material for top electrode 22, pad structure 22' and trace 25 may also include deposited metals such as silver, gold, etc. The top electrode 22, pad structure 22' and the trace 25 may have a thickness of less than one micron to a few microns.

A dielectric layer 27 (transparent in FIG. 1A) is applied over the top electrode 22 and trace 25. The dielectric layer 27 may be applied by dipping the needle 14, by masked deposition, by painting or by another process. The dielectric prevents shorting against a trace 24 to be formed.

A top electrode 23, pad structure 23' and the signal trace 24 connecting the top electrode 23 to the pad structure 23' are formed for the sensor 10. The top electrode 23 is formed over the copolymer 20 at the distal end portion, and the trace 24 and pad structure 23' are formed over a portion of the insulator 16 (and/or dielectric layer 27). The needle 14 forms the bottom electrode. The top electrode 23, pad structure 23' and the trace 24 may be printed using a conductive ink. Other processes may be employed as well such as, e.g., masked vapor deposition or vapor deposition and etching. The material for top electrode 23, pad structure 23' and trace 24 may also include deposited metals such as silver, gold, etc. The top electrode 23, pad structure 23' and the trace 24 may have a thickness of less than one micron to a few microns.

Another insulator 26 is preferably formed over the trace 25 and insulator layer 16. This insulator 26 may be produced by dip coating from the proximal end of the needle 14. The insulator 26 is deposited or printed on the needle 14. The insulator 26 may include any suitable dielectric material that adheres to underlying materials. The insulator 26 may be about 25 - 50 microns thick although other thicknesses may be employed.

Another insulator 31 is preferably formed over the trace 24 and insulator layer 26. This insulator 31 may be produced by dip coating from the proximal end on the needle 14. The insulator 31 is deposited or printed on the needle 14. The insulator 31 may include any suitable dielectric material that adheres to underlying materials. The insulator 31 may be about 25 - 50 microns thick although other thicknesses may be employed.

A conductive shield 28 may be applied over the insulator 31 in regions where the traces 24, 25 are present. The conductive shield 28 may be produced by vapor deposition or dip coating in conductive ink. Care needs to be taken to not cover the tip (distal end portion of the needle 14). The needle 14 and outer shield 28 will be coupled together as they form a driven shield. To electrically insulate the top electrodes 22, 23 from the surroundings and ensure biocompatibility, the whole needle could be covered with, for example, parylene or other outer dielectric material 29. By properly selecting acoustic properties and thickness, the outer dielectric material 29 may serve as an acoustic matching layer.

For the dielectric layers, e.g., insulator 16, 26 and the outer dielectric, it is advantageous to select a material with a relatively low dielectric constant. For example, polytetrafluoroethylene (PTFE) with a dielectric constant of about 2.1 may be selected. However, the adhesion of PTFE to other materials may be an issue. Other materials, such as biocompatible polypropylene (dielectric constant 2.2) may be employed. Many plastics/polymers have a dielectric constant close to 3.0 and may also be employed. Polyurethane has a slightly higher 3.5 value and is attractive for use in the present applications because there are medical grade versions (used to coat implantable pacemakers). Further, polyurethane provides good adhesion to many materials with high smoothness and durability, and can be deposited in thin layers using appropriate solvents. Other materials may also be used.

The sensor 10 forms a ring shaped sensor and electrical interconnect or trace 24 on the needle 14. Likewise, the sensor 11 is also applied with a structure similar in geometry to sensor 10 and in close proximity to sensor 10. Sensor 11 is not sensitive to ultrasound (for example, sensor 11 includes a non-poled (or non-piezoelectric) sensor with interconnect or trace 25. Discrimination of a strong acoustic event from outside interference may be provided by observing the strong signal only on sensor 10 or on both sensor 10 and 11.

The single ring sensor 10 provides maximum sensor sensitivity due to a strong impedance difference between the sensor 10 and its backing material. The narrow trace 24 is provided and minimizes the capacitive loading of the sensor 10. The thin interconnect trace 24 may be shielded similarly to a stripline configuration to be optimized for low capacitance. The sensor 10 can be more sensitive to injected noise as the needle 14 is in electrical contact with tissue (when filled with fluid or stylet), which is part of the interconnect.

The sensor 10 may include a P(VDF-TrFE) copolymer ring 20 shaped onto the needle 14. The ring contact pad structure 22' is formed at a hub end (proximal end portion) and provides for low disposable cost connectivity. Specialized electronics can be provided to reduce signal loss due to capacitive loading of the interconnect.

The present principles can be extended to multiple sensors on a same needle. This permits a determination of orientation of the needle and also determination of the location of the needle tip without the need to place the sensor very close to the tip. Calculating the tip location based on signals from multiple sensors should also increase the measurement accuracy as well as provide an indication of confidence in the measurement. The cost is a slightly more complicated manufacturing process and a slight loss of signal because of the extra capacitive load of multiple sensors.

If the sensor material under the top electrode 23 is made out of piezoelectric poled P(VDF-TrFE) (20), non-poled P(VDF-TrFE) (21) may be employed for the dummy sensor 11. If it is desired to fine tune the response of the dummy sensor 11 to more closely match noise signals from the sensor 10, the non-poled P(VDF-TrFE) layer 21 may be made thinner so it matches the capacitance of the poled P(VDF-TrFE) layer 20 in the sensor 10 (poling process leads to a higher capacitance). One could also use a different dielectric material (21) for the dummy sensor 11 to match the capacitance of sensor 10.

It should be understood that different numbers of dielectric layers, traces and other structures may be employed. These structures may be configured in different arrangements as needed to provide a sensor(s) and a corresponding dummy structure(s).

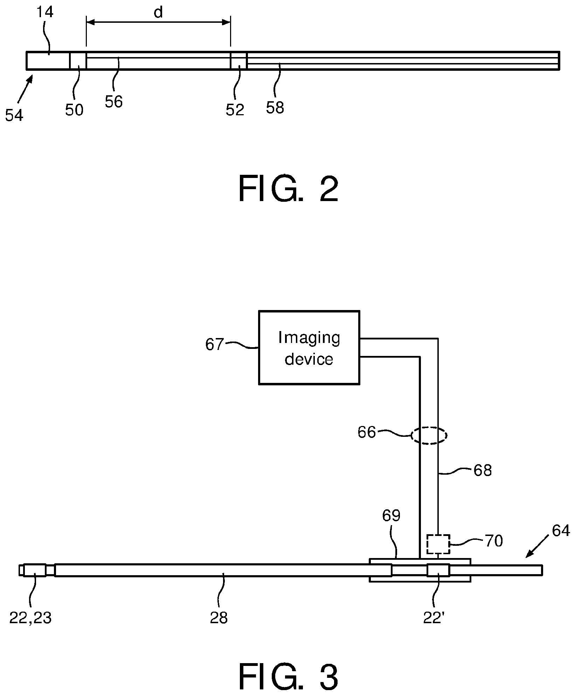

Referring to FIG. 2, in another embodiment, two ring sensors 50 and 52 may be employed to provide multiple sensors on a needle 14. This permits the determination of orientation of the needle 14 as well as the determination of the location of a needle tip 54 without the need to place a sensor very close to the tip. Calculating the tip location based on signals from multiple sensors 50, 52 also increases the measurement accuracy as well as provides an indication of confidence in the measurement. When two sensors are placed on the needle with sufficient separation between them (e.g., at least a few mm), one sensor can act as the dummy sensor for the other sensor. The noise signal that is picked up (from a non-acoustic event) is largely due to the interconnect structure (traces) acting as an antenna, and its behavior is dominated by the shape of the long interconnect trace (the smaller dimensioned ring electrodes will have much less influence on the noise behavior). When two sensors 50, 52 are placed with sufficient separation on the needle and a focused ultrasound beam from an ultrasound imaging probe creates a strong acoustic generated signal in the needle 14, it can be assumed that a focal spot is very near to one of the sensors and further from the other sensor. Thus, an acoustically generated strong signal should only be observed in one of the sensors, the other sensor will at best pick up a much weaker signal from an acoustic side lobe that, in most cases, will also not occur at the same time point within a frame.

If the two sensors 50, 52 have individual traces 56, 58 bringing out the signals, and the traces 56, 58 are made in close proximity and with similar geometry, the non-acoustic noise signals picked up by these traces 56, 58 should be very similar in strength and timing. To create similar traces in, for example, a needle with two sensors 52, 54 that are spaced a distance d apart (e.g., about 1 cm apart, although other distances are contemplated) with one sensor near the tip, the interconnect traces 56, 58 would be closely spaced running in parallel and both running a long distance down the needle 14. One trace would then be connected to the distal sensor electrode and the other to the proximal sensor electrode. The minimum distance between the sensors 50, 52 should exceed a resolution cell of the ultrasound imaging system, for one sensor to act as the dummy signal for the other sensor.

It should be understood that the embodiments described in FIGS. 1A-D and 2 may be extended to three or more sensors. In this case, all the interconnect traces could run parallel to each other (or some stacked on top of each other in multilayer implementation). More sophisticated noise rejection schemes may be employed in such an arrangement. With all sensor data for an ultrasound transmit frame collected, needle orientation may be estimated based on the strongest received signals (and assuming the signals all have an acoustic origin). Based on the estimated orientation and knowledge about imaging beam sidelobes, predictions as to what times some acoustic signal should be detected by the different sensors could be made and at what times no acoustic signals should be present. Making certain assumptions about tissue properties, the expected amplitude of the signals can be modeled due to acoustic insonification for all sensors and time points (also taking into account the angle dependent sensitivity of the sensors). Also, very strong signals with amplitude that far exceeds the maximum achievable amplitude due to an acoustic event (this depends on transducer, ultrasound machine settings, and perceived depth of the strong signal) can be outright ignored in the analysis.

Based on the estimated modeling of the received signal, inconsistencies in the received signal may be employed to reject tracking data that falls below a certain confidence level. With sufficient computational resources, an iterative procedure may be employed where the most inconsistent received signal is removed because it is assumed to come from a non-acoustic origin, and the estimated orientation is gradually refined over multiple noise removal cycles.

A beam pattern of a sensor is used to transmit a beam profile, given an initial estimated needle orientation, and with assumed tissue attenuation properties, model the expected received amplitude in time. This expected received amplitude can be compared with the real amplitude, and a larger mismatch decreases confidence in either the estimated needle position or the contribution of this particular sensor to achieving an accurate needle orientation. As this is done for multiple sensors, a large mismatch for all sensors indicates the estimated needle position is incorrect and should be discarded. A large mismatch in only one sensor indicates that sensor has a corrupting signal and analysis should be repeated without this particular sensor contribution.

In other embodiments, sensors may be deposited on or otherwise attached directly onto a needle (or other device). For example, the sensors may be deposited on an insulating layer such that a bottom electrode and a top electrode contact a piezo-electric material (or non-poled piezoelectric material for a dummy sensor) over the insulating layer (e.g., the needle no longer being the bottom electrode). The traces for both top and bottom electrodes are employed for each sensor.

Alternatively, an insulating layer can be deposited on the needle, on top of that a conductive layer that forms the bottom electrode of all sensors, and bring out separate traces for the top electrodes. The bottom electrode could thereby be shared for all sensors (either a separate layer or through the needle) and connect several of the top electrodes to one mutual trace (and other subsets of electrodes to other mutual traces).

In other embodiments, balanced signal sensors may be constructed that have three leads each: a reference, a positive, and a negative lead. Balanced signal sensors could share some of the leads in combinations with sensor subsets. Sensors can have a different geometry than the ring shape described in above embodiments, for example, patches may be employed that circumvent less than the full circumference of the needle. Traces do not have to be straight and could be, for example, on different layers and shaped to resemble twisted pairs. The different embodiments described herein may be combined in a multitude of possible combinations.

In still other embodiments, a multitude of real sensors, a multitude of dummy sensors, and a multitude of dummy traces may be employed. To determine if a strong signal that is received on a real sensor is due to the imaging beam hitting the sensor, one could see, if around the same time, a signal is observed on nearby dummy sensors. If there is such a signal then the event can be determined to be external noise from a non-acoustic source. Next, the signals on the other dummy sensors may be observed, and interpreted the same way. The signal on the dummy traces may be observed and interpreted in the same way. The signals on other real sensors that are at least an ultrasound resolution cell away may be observed. If strong signals around the same time are observed, this indicates an acoustic origin signal from a non-focused source (such as a reverb or plane wave emission) and this signal should not be used for tracking.

If the detected signal is estimated to be from a true ultrasound (US) signal source, then the signal is processed and displayed as usual. If the detected signal is estimated to be emanating from a noise source, then that signal is not used in subsequent processing. Alternatively, a message may be displayed to the user indicating that noise is being detected. Corrective actions such as identifying and shutting down error sources may be identified and acted on by the user.

Referring to FIG. 3, another dummy structure may also be formed by adding an extra wire 68 in a connecting cable 66. A needle 64 (or other device) is connected to the cable 66. The cable 66 connects to hub contacts (e.g., contacts 22', 23', shield 28 and the needle 64 via an interconnect 69. In this embodiment, the cable 66 includes the extra wire 68 that does not connect to a structure on the needle itself (as most of the noise pick up may take place in the connecting cable).

As before, if the detected signal is estimated to be from a true US signal source, then the signal is processed and displayed as usual. If the detected signal is estimated to be emanating from a noise source, then that signal is not used in subsequent processing.

In another embodiment, the wire 68 may include a dummy sensor 70 at the end of the cable 66 that connects to the needle 64. The noise is picked up by the combination of wires in the cable 66 that connect the medical device 64 to an imaging system 67, the interconnect 69 that connects the end of the cable 66 to the sensors on the medical device 64, and the sensors (23, and optionally dummy sensors 22) on the medical device 64. If the dominant noise pickup takes place in the connecting cable it may be sufficient to have a dummy structure in the cable portion of the system only without the need for a dummy sensor on the 64 (or other device).

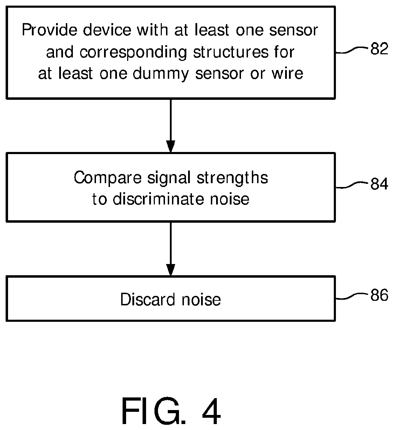

Referring to FIG. 4, a method for discriminating between signal and noise measured by a medical device is illustrative shown. In block 82, a medical device is provided having a device body and at least one first sensor formed on the device body and including a piezoelectric polymer as a sensor element. The piezoelectric polymer is configured to receive ultrasonic energy. At least one first electrical trace connects to the at least one first sensor and extends along the device body. At least one dummy sensor is formed on the device body in proximity of the at least one first sensor and includes a dummy sensor element. At least one second electrical trace connects to the at least one dummy sensor and extends along the device body in a configuration relative to the at least one first electrical trace. Other dummy noise components may also be employed, e.g., dummy sensors not on the medical device, dummy wires, etc.

In block 84, signal strength measured for first sensors, dummy sensors, second electrical traces, etc. is compared to determine whether a candidate signal is signal or noise. This includes finding a strongest signal and comparing it to other signals to determine whether other signals are stronger and represent an actual signal or noise.

In one embodiment, the real sensor(s) (e.g., P(VDF-TrFE) ring shaped sensor and electrical interconnect on the needle), and dummy sensor(s), which has a structure similar in geometry to its corresponding real sensor and is in close proximity to the real sensor measure an event. The dummy sensor is not sensitive to ultrasound; for example, it includes an unpoled (non-piezoelectric) sensor with interconnect. Discrimination of a strong acoustic event from outside interference can be performed by observing strong signal only on the real sensor or on both real sensor and the dummy sensor.

First, strong signals (a local maximum) on the real sensor are identified and compared to signal levels shortly preceeding and shortly after the event to obtain a rough signal to noise ratio (SNR) estimate of the suspected acoustic event. Next, an SNR estimate for the exact same time windows is performed on the dummy sensor, and when the dummy sensor has an SNR that, for example, exceeds the threshold of the real sensor (e.g., SNR -10dB) the signal is assumed to not originate from an acoustic event and is thus disregarded in tracking calculations.

In block 86, noise signals are discarded or disregarded for further image processing. This is achieved by eliminating the weaker or noncontributing signals and using the stronger confirmed signals for imaging or locating a device, etc.

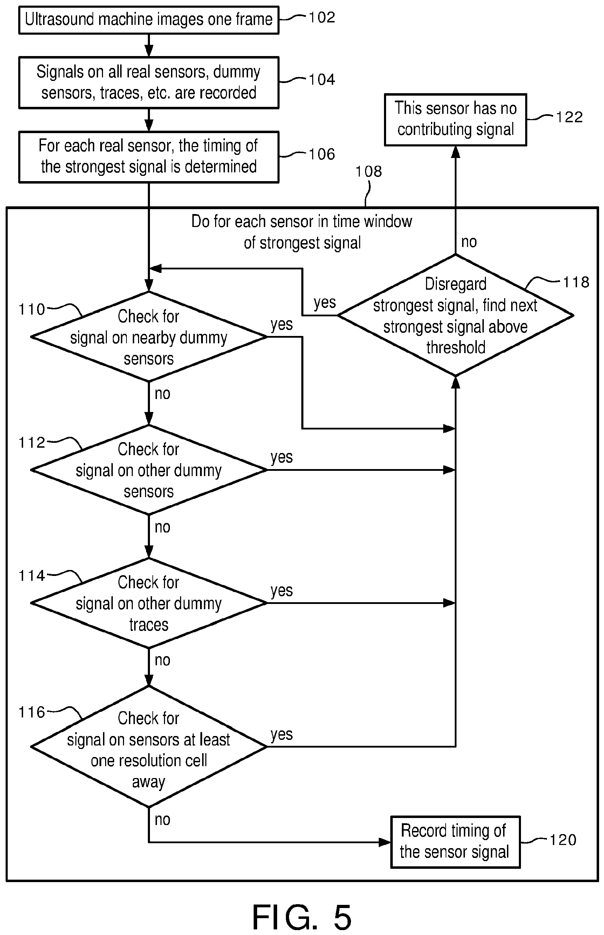

Referring to FIG. 5, a block/flow diagram shows a system/method for isolating relevant sensor signals resulting from one imaging frame for use in imaging or for estimating a needle orientation, for the case of a needle with a multitude of sensors, dummy sensors, and dummy traces. The following method more specifically defines how signal strength is compared and analyzed in block 84 of FIG. 3. In block 102, an ultrasonic machine images one frame. In block 104, signals on all real sensors, dummy sensors and dummy traces are recorded. In block 106, for each real sensor, the timing of a strongest signal is determined. In block 108, when a strong signal is detected on a real sensor, the following steps are performed to check if the signal is coming from an imaging beam. This is done by checking for the absence of a strong signal in the same time frame on other sources that are not picking up an imaging beam related signal but can pick up noise.

In block 110, the signal on nearby dummy sensors (and wires, etc.) is checked to determine if it is close in strength (for example less then 10dB weaker) or stronger than the signal of the real sensor. If it is close in strength or stronger then the path goes to block 118. Otherwise, the path goes to block 112. In block 112, the signal on other dummy sensors is checked to determine if it is comparable or stronger than the signal of the real sensor. If it is comparable or stronger, then the path goes to block 118. Otherwise, the path goes to block 114. In block 114, the signal on dummy traces is checked to determine if it is stronger than the signal of the real sensor. If it is stronger, then the path goes to block 118. Otherwise, the path goes to block 116. In block 116, the signal on real sensors at least one resolution cell distance away is checked to determine if it is very close in strength (for example less than 6dB weaker) or stronger than the signal of the real sensor. If it is very close or stronger, then the path goes to block 118. Otherwise, the path goes to block 120 where the timing of the real sensor signal is recorded. The nearby dummy sensors are checked first, as trace and sensor geometry are closest and thus it is most likely that they will pick up similar noise signals. Next, the other dummy sensors are checked as well as the dummy traces.

In block 118, since comparable or stronger signals than the real sensor signal were found, the respective candidate signals can be disregarded. This is done in order of most significance such that if presence of one additional signal is detected no further processing time is spent on checking the other sources. To not loose tracking in the presence of frequent noise signals, the decision box of block 118 repeats the analysis for the next strongest signal if it is above a certain threshold. If it is not above this threshold, the sensor is deemed to have no contribution in block 122.

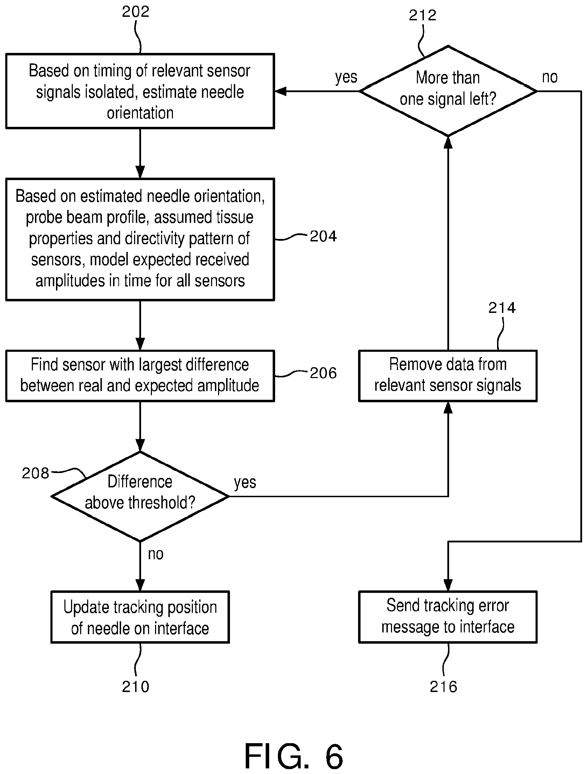

Referring to FIG. 6, the output of FIG. 5 can form the input for a tracking method of the block/flow diagram of FIG. 5. In FIG. 6, an iterative procedure for refining the needle (or device) position or determining that the needle position estimate is not reliable is provided. Based on all the relevant sensor signals, an estimate is made of the needle (or device) orientation, in block 202, based on the timing information from block 120 (FIG. 5). In block 204, based on the estimated needle orientation, probe beam properties, and directivity patterns of the sensors, model expected received amplitudes in time for all sensors. According to the estimated needle orientation, the expected signals on all the real sensors can be modeled. The sensor signal that deviates most from the predicted signal is a potential outlier that has detrimental influence on the estimate. This is determined in block 206.

In block 208, if the difference of sensor signal that deviates most between real and expected amplitude exceeds a threshold, the sensor signal is removed from the data set in block 214. If the difference is small in block 208, the orientation estimate is considered valid. In block 210, the position of the needle or other device is updated on a user interface (e.g., display). In block 212, if after removal of the dataset (block 214) less than two signals remain to be processed, a needle orientation estimate cannot be made and a tracking error message is sent to the interface in block 216.

The present principles have been described in terms of a needle, and more particularly to a biopsy needle. However, the present principles may be applied to any instrument where a piezoelectric sensor (receiver), transmitter or transducer is needed. Such devices may include catheters, guidewires, endoscopes, implantable devices, etc. The present principles can provide a relatively low cost device with a built-in for sensor conformally applied to an exterior surface. To keep the product cost down, the materials used need to be low cost, and the manufacturing process should be highly automated with large volume to avoid labor and equipment cost. The devices in accordance with the present principles provide a low form factor that is conformally formed and placed on a medical device or instrument. In particularly useful embodiments, the present principles are employed for ultrasound guided needle interventions, e.g., RF ablation, liver biopsy, nerve blocks, vascular access, abscess drainage, etc.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for signal versus noise discrimination needle with piezoelectric polymer sensors (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims.

## Claims

1. A medical device, comprising:
a device body (14);
a first sensor (10) formed on the device body and including a piezoelectric polymer as a sensor element, the piezoelectric polymer configured to detect ultrasonic energy, and a first electrical trace (24) connecting to the first sensor element and extending along the device body; and
a dummy sensor (11) formed on the device body in proximity of the first sensor and including a dummy sensor element that is insensitive to ultrasound, and a second electrical trace (25) connecting to the dummy sensor element and extending along the device body in a configuration relative to the first electrical trace, wherein the first sensor and the dummy sensor are arranged for discriminating a signal event between a signal and noise based on a comparison between a signal strength measured on the first sensor and the dummy sensor.

2. The medical device as recited in claim 1, wherein the piezoelectric polymer (20) includes one of polyvinylidene fluoride (PVDF) or polyvinylidene fluoride trifluoroethylene P(VDF-TrFE).

3. The medical device as recited in claim 1, wherein the dummy sensor element (21) includes one of non-poled piezo-electric material or a dielectric material.

4. The medical device as recited in claim 1, further comprising a conductive shield (28) formed over the first and second electrical traces.

5. The medical device as recited in claim 1, wherein the configuration relative to the first electrical trace includes the first and second traces running parallel to one another.

6. The medical device as recited in claim 1, wherein the signal event includes a signal generated by an acoustic source and the noise includes radio frequency interference.

7. The medical device as recited in claim 1, wherein the medical device includes a plurality of first sensors (50, 52) with first electrical traces and a plurality of corresponding dummy sensors with second electrical traces for discriminating the signal event based upon a measurement at positions of one or more first sensors and dummy sensors.

8. The medical device as recited in claim 1, wherein the medical device includes a plurality of first sensors (50, 52) for determining an orientation of the medical device based upon measurements made by the plurality of first sensors.

9. The medical device as recited in claim 1 wherein:
the device body (14) comprises a needle;
the first sensor comprises a ring sensor (10) conformally formed on the needle and including a piezoelectric polymer as a sensor element, the piezoelectric polymer being configured to detect ultrasonic energy;
a first dielectric layer (16) is formed on the needle;
the first electrical trace (24) extends longitudinally along the needle on the first dielectric layer;
the dummy sensor (11) has the same geometry as the first sensor; and
the second electrical trace (25) extends longitudinally along the needle parallel to the first electrical trace.

10. The medical device as recited in claim 9, further comprising a second dielectric layer formed over the first and second electrical traces and a conductive shield (28) formed on the second dielectric layer over the first and second electrical traces.

11. The medical device as recited in claim 9, wherein the device includes a plurality of first sensors (50, 52) with first electrical traces and a plurality of corresponding dummy sensors with second electrical traces for discriminating a signal event between a signal and noise based on a comparison between a response measured at positions of one or more first sensors and positions of one or more dummy sensors.

12. A method for discriminating between signal and noise measured by a medical device, comprising:
providing (82) a medical device having a device body; at least one first sensor formed on the device body and including a piezoelectric polymer as a sensor element, the piezoelectric polymer being configured to detect ultrasonic energy; the at least one first sensor comprising at least one first electrical trace connecting to the first sensor element and extending along the device body, at least one dummy sensor formed on the device body in proximity of the at least one first sensor and including a dummy sensor element that is insensitive to ultrasound; the at least one dummy sensor comprising at least one second electrical trace connecting to the dummy sensor element and extending along the device body in a configuration relative to the at least one first electrical trace;
comparing (84) signal strength measured for first sensors and dummy sensors to determine whether a candidate signal is signal or noise; and
discarding (86) noise signals.

13. The method as recited in claim 12, wherein comparing signal strength includes:
for a strongest candidate signal measured from the at least one first sensor, checking (110, 112, 114) signal strength measured for first sensors and dummy sensors to determine whether the candidate signal is strongest; and
if the strongest candidate signal is approximated or exceeded by a signal from another first sensor and dummy sensor, disregarding (118) a previous strongest candidate signal in favor of another strong signal measured on the at least one first sensor.

14. The method as recited in claim 13, further comprising:
estimating (202) an orientation of the medical device based upon timing of first sensor signals;
modeling (204) expected amplitudes of detected signals using the orientation estimated for the medical device;
for a first sensor with a largest difference between expected amplitude and measured amplitude, determining (208) whether the difference is greater than a threshold;
if the threshold is exceeded, removing (214) the signal from consideration;
if the threshold is not exceeded, updating (210) a tracked position of the medical device in accordance with the first sensor with a largest difference.

15. The method as recited in claim 13, wherein checking signal strength measured for first sensors includes checking (116) first sensors at least one resolution cell away for an ultrasonic imaging cell.

## Patentansprüche

1. Medizinische Vorrichtung, umfassend:
einen Vorrichtungskörper (14);
einen ersten Sensor (10), der auf dem Vorrichtungskörper ausgebildet ist und ein piezoelektrisches Polymer als Sensorelement enthält, wobei das piezoelektrische Polymer konfiguriert ist, um Ultraschallenergie zu erfassen, und eine erste elektrische Spur (24), die mit dem ersten Sensorelement verbunden ist und sich entlang des Vorrichtungskörpers erstreckt; und
einen Dummy-Sensor (11), der auf dem Vorrichtungskörper in der Nähe des ersten Sensors ausgebildet ist und ein Dummy-Sensorelement enthält, das gegenüber Ultraschall unempfindlich ist, und eine zweite elektrische Spur (25), die mit dem Dummy-Sensorelement verbunden ist, und sich entlang des Vorrichtungskörpers in einer Konfiguration relativ zur ersten elektrischen Spur erstreckt, wobei der erste Sensor und der Dummy-Sensor angeordnet sind, um ein Signalereignis zwischen einem Signal und Rauschen basierend auf einem Vergleich zwischen einer am ersten Sensor gemessenen Signalstärke und dem Dummy-Sensor zu unterscheiden.

2. Medizinische Vorrichtung nach Anspruch 1, wobei das piezoelektrische Polymer (20) eines aus Polyvinylidenfluorid (PVDF) oder Polyvinylidenfluoridtrifluorethylen P(VDF-TrFE) enthält.

3. Medizinische Vorrichtung nach Anspruch 1, wobei das Dummy-Sensorelement (21) eines aus nicht gepoltem piezoelektrischem Material oder einem dielektrischen Material enthält.

4. Medizinische Vorrichtung nach Anspruch 1, ferner umfassend eine leitende Abschirmung (28), die über der ersten und zweiten elektrischen Spur ausgebildet ist.

5. Medizinische Vorrichtung nach Anspruch 1, wobei die Konfiguration in Bezug auf die erste elektrische Spur die erste und die zweite Spur umfasst, die parallel zueinander verlaufen.

6. Medizinische Vorrichtung nach Anspruch 1, wobei das Signalereignis ein von einer Schallquelle erzeugtes Signal enthält und das Rauschen Hochfrequenzstörungen enthält.

7. Medizinische Vorrichtung nach Anspruch 1, wobei die medizinische Vorrichtung mehrere erste Sensoren (50, 52) mit ersten elektrischen Spuren und mehrere entsprechende Dummy-Sensoren mit zweiten elektrischen Spuren zum Unterscheiden des Signalereignisses basierend auf einer Messung an Positionen eines oder mehrerer erster Sensoren und Dummy-Sensoren enthält.

8. Medizinische Vorrichtung nach Anspruch 1, wobei die medizinische Vorrichtung mehrere erste Sensoren (50, 52) zum Bestimmen einer Orientierung der medizinischen Vorrichtung auf der Grundlage von Messungen enthält, die von mehreren ersten Sensoren durchgeführt werden.

9. Medizinische Vorrichtung nach Anspruch 1, wobei:
der Vorrichtungskörper (14) eine Nadel umfasst;
der erste Sensor einen Ringsensor (10) umfasst, der konform auf der Nadel ausgebildet ist und ein piezoelektrisches Polymer als Sensorelement enthält, wobei das piezoelektrische Polymer konfiguriert ist, um Ultraschallenergie zu erfassen;
eine erste dielektrische Schicht (16) auf der Nadel ausgebildet ist;
die erste elektrische Spur (24) sich in Längsrichtung entlang der Nadel auf der ersten dielektrischen Schicht erstreckt;
der Dummy-Sensor (11) die gleiche Geometrie wie der erste Sensor aufweist; und
die zweite elektrische Spur (25) sich in Längsrichtung entlang der Nadel parallel zur ersten elektrischen Spur erstreckt.

10. Medizinische Vorrichtung nach Anspruch 9, ferner umfassend eine zweite dielektrische Schicht, die über der ersten und zweiten elektrischen Spur ausgebildet ist, und eine leitende Abschirmung (28), die auf der zweiten dielektrischen Schicht über der ersten und zweiten elektrischen Spur ausgebildet ist.

11. Medizinische Vorrichtung nach Anspruch 9, wobei die Vorrichtung mehrere erste Sensoren (50, 52) mit ersten elektrischen Spuren und mehrere entsprechende Dummy-Sensoren mit zweiten elektrischen Spuren zum Unterscheiden eines Signalereignisses zwischen einem Signal und Rauschen enthält, basierend auf einem Vergleich zwischen einer Rückmeldung, die an die Positionen eines oder mehrerer erster Sensoren und Positionen eines oder mehrerer Dummy-Sensoren gemessen wurde.

12. Verfahren zur Unterscheidung zwischen Signal und Rauschen, das von einer medizinischen Vorrichtung gemessen wird, umfassend:
Bereitstellen (82) einer medizinischen Vorrichtung die einen Vorrichtungskörper aufweist; mindestens ein erster Sensor, der auf dem Vorrichtungskörper ausgebildet ist und ein piezoelektrisches Polymer als Sensorelement enthält, wobei das piezoelektrische Polymer so konfiguriert ist, dass es Ultraschallenergie erfasst, wobei der mindestens erste Sensor mindestens eine erste elektrische Spur umfasst, die mit dem ersten Sensorelement verbunden ist und sich entlang des Gerätekörpers erstreckt; mindestens einen Dummy-Sensor, der auf dem Vorrichtungskörper in der Nähe des mindestens einen ersten Sensors ausgebildet ist, und ein Dummy-Sensorelement enthält, das gegenüber Ultraschall unempfindlich ist; wobei der mindestens eine Dummy-Sensor mindestens eine zweite elektrische Spur umfasst, die mit dem Dummy-Sensorelement verbunden ist und sich entlang des Vorrichtungskörpers in einer Konfiguration relativ zu der mindestens einen ersten elektrischen Spur erstreckt;
Vergleichen (84) der für erste Sensoren und Dummy-Sensoren gemessenen Signalstärke, um festzustellen, ob ein Kandidatensignal ein Signal oder ein Rauschen ist; und
Verwerfen von (86) Rauschsignalen.

13. Verfahren nach Anspruch 12, wobei das Vergleichen der Signalstärke umfasst:
für ein stärkstes Kandidatensignal, das von dem mindestens einen ersten Sensor gemessen wird, das Überprüfen der (110, 112, 114) Signalstärke, die für erste Sensoren und Dummy-Sensoren gemessen wurde, um zu bestimmen, ob das Kandidatensignal ist am stärksten; und
wenn das stärkste Kandidatensignal durch ein Signal von einem anderen ersten Sensor und einem Dummy-Sensor angenähert oder überschritten wird, Ignorieren (118) ein vorheriges stärkstes Kandidatensignal zugunsten eines anderen starken Signals, das an dem mindestens einen ersten Sensor gemessen wurde.

14. Verfahren nach Anspruch 13, ferner umfassend:
Schätzen (202) einer Orientierung der medizinischen Vorrichtung basierend auf dem Zeitpunkt der ersten Sensorsignale;
Modellierung (204) erwarteter Amplituden detektierter Signale unter Verwendung der für das Medizinprodukt geschätzten Orientierung;
für einen ersten Sensor mit der größten Differenz zwischen der erwarteten Amplitude und der gemessenen Amplitude, Bestimmen (208), ob die Differenz größer als ein Schwellenwert ist;
wenn der Schwellenwert überschritten wird, Entfernen (214) das Signal aus der Betrachtung;
wenn der Schwellenwert nicht überschritten Aktualisieren (210) eine verfolgte Position des medizinischen Geräts gemäß dem ersten Sensor mit dem größten Unterschied.

15. Verfahren nach Anspruch 13, wobei das Überprüfen der für erste Sensoren gemessenen Signalstärke das Überprüfen (116) der ersten Sensoren, die mindestens eine Auflösungszelle entfernt sind, für eine Ultraschallbildgebungszelle umfasst.

## Revendications

1. Dispositif médical, comprenant:
un corps de dispositif (14);
un premier capteur (10) formé sur le corps du dispositif et comprenant un polymère piézoélectrique comme élément capteur, le polymère piézoélectrique configuré pour détecter l'énergie ultrasonore, et une première trace électrique (24) se connectant au premier élément capteur et s'étendant le long du corps du dispositif; et
un capteur factice (11) formé sur le corps du dispositif à proximité du premier capteur et comprenant un élément capteur factice qui est insensible aux ultrasons, et une deuxième trace électrique (25) se connectant à l'élément capteur factice et s'étendant le long du corps du dispositif dans une configuration relative à la première trace électrique, où le premier capteur et le capteur factice sont agencés pour discriminer un événement de signal entre un signal et un bruit sur la base d'une comparaison entre une force de signal mesurée sur le premier capteur et le capteur factice.

2. Dispositif médical selon la revendication 1, dans lequel le polymère piézoélectrique (20) comprend un polyfluorure de vinylidène (PVDF) ou un poly(fluorure de vinylidène-trifluoréthylène) P(VDF-TrFE).

3. Dispositif médical selon la revendication 1, dans lequel l'élément capteur factice (21) comprend un matériau piézo-électrique non polaire ou un matériau diélectrique.

4. Dispositif médical selon la revendication 1, comprenant en outre un blindage conducteur (28) formé sur les première et deuxième traces électriques.

5. Dispositif médical selon la revendication 1, dans lequel la configuration relative à la première trace électrique comprend les première et deuxième traces qui s'étendent parallèles l'une à l'autre.

6. Dispositif médical selon la revendication 1, dans lequel l'événement de signal comprend un signal généré par une source acoustique et le bruit comprend une interférence de radiofréquences.

7. Dispositif médical selon la revendication 1, dans lequel le dispositif médical comprend une pluralité de premiers capteurs (50, 52) avec des premières traces électriques et une pluralité de capteurs factices correspondants avec des secondes traces électriques pour discriminer l'événement de signal sur la base d'une mesure à des positions d'un ou plusieurs premiers capteurs et capteurs factices.

8. Dispositif médical selon la revendication 1, dans lequel le dispositif médical comprend une pluralité de premiers capteurs (50, 52) pour déterminer une orientation du dispositif médical sur la base de mesures effectuées par la pluralité de premiers capteurs.

9. Dispositif médical selon la revendication 1, dans lequel:
le corps de dispositif (14) comprend une aiguille;
le premier capteur comprend un capteur annulaire (10) formé conformément à l'aiguille et comprenant un polymère piézoélectrique comme élément capteur, le polymère piézoélectrique étant configuré pour détecter l'énergie ultrasonore;
une première couche diélectrique (16) est formée sur l'aiguille;
la première trace électrique (24) s'étend longitudinalement le long de l'aiguille sur la première couche diélectrique;
le capteur factice (11) a la même géométrie que le premier capteur; et
la deuxième trace électrique (25) s'étend longitudinalement le long de l'aiguille parallèlement à la première trace électrique.

10. Dispositif médical selon la revendication 9, comprenant en outre une deuxième couche diélectrique formée sur les première et deuxième traces électriques et un blindage conducteur (28) formé sur la deuxième couche diélectrique sur les première et deuxième traces électriques.

11. Dispositif médical selon la revendication 9, dans lequel le dispositif comprend une pluralité de premiers capteurs (50, 52) avec des premières traces électriques et une pluralité de capteurs factices correspondants avec des deuxièmes traces électriques pour discriminer un événement de signal entre un signal et un bruit sur la base d'une comparaison entre une réponse mesurée aux positions d'un ou plusieurs premiers capteurs et les positions d'un ou plusieurs capteurs factices.

12. Procédé pour discriminer le signal et le bruit mesuré par un dispositif médical, comprenant:
la fourniture (82) d'un dispositif médical ayant un corps de dispositif; au moins un premier capteur formé sur le corps du dispositif et comprenant un polymère piézoélectrique comme élément capteur, le polymère piézoélectrique étant configuré pour détecter l'énergie ultrasonore; l'au moins un premier capteur comprenant au moins une première trace électrique se connectant au premier élément capteur et s'étendant le long du corps du dispositif; au moins un capteur factice formé sur le corps du dispositif à proximité d'au moins un premier capteur et comprenant un élément capteur factice qui est insensible aux ultrasons; l'au moins un capteur factice comprenant au moins une seconde trace électrique se connectant à l'élément capteur factice et s'étendant le long du corps du dispositif dans une configuration par rapport à l'au moins une première trace électrique;
la comparaison (84) de la force du signal mesurée pour les premiers capteurs et les capteurs factices pour déterminer si un signal candidat est un signal ou un bruit; et
le rejet (86) des signaux de bruit.

13. Procédé selon la revendication 12, dans lequel la comparaison de la force du signal comprend:
pour un signal candidat le plus fort mesuré à partir du au moins un premier capteur, la vérification (110, 112, 114) de la force du signal mesurée pour les premiers capteurs et les capteurs factices pour déterminer si le signal candidat est le plus fort; et
si le signal candidat le plus fort est approché ou dépassé par un signal provenant d'un autre premier capteur et capteur factice, ignorer (118) un signal candidat précédent le plus fort en faveur d'un autre signal fort mesuré sur l'au moins un premier capteur.

14. Procédé selon la revendication 13, comprenant en outre:
l'estimation (202) d'une orientation du dispositif médical basée sur un moment des premiers signaux de capteur;
la modélisation (204) des amplitudes attendues des signaux détectés en utilisant l'orientation estimée pour le dispositif médical;
pour un premier capteur avec la plus grande différence entre l'amplitude attendue et l'amplitude mesurée, le déterminer (208) si la différence est supérieure à un seuil;
si le seuil est dépassé, retirer (214) de la considération le signal;
si le seuil n'est pas dépassé, mettre à jour (210) une position suivie du dispositif médical conformément au premier capteur avec une plus grande différence.

15. Procédé selon la revendication 13, dans lequel la vérification de la force du signal mesurée pour les premiers capteurs comprend la vérification (116) des premiers capteurs à au moins une cellule de résolution de distance pour une cellule d'imagerie ultrasonique.
